# EUROPEAN PATENT APPLICATION

(11) **EP 4 759 191 A1**
(43) Date of publication of application: **17.06.2026**
(21) Application number: 25222163.5
(22) Date of filing: 10.12.2025
(51) Int. Cl.: A45D 33/00, A45D 33/18, A45D 40/00, A61K 8/02

(54) **A METHOD FOR MAKING A COSMETIC PRODUCT**

(30) Priority: 13.12.2024 IT 202400028395
(71) Applicant: Chromavis S.p.A., 26010 Offanengo (CR) (IT)
(72) Inventor: Sangalli, Andrea Rubens, 26010 Campagnola Cremasca (CR) (IT); Uggè, Martina, 26012 Castelleone (CR) (IT)
(74) Representative: Vanosi, Adelio Valeriano

(57) **Abstract**

A method for making a cosmetic product (1) comprising a plate-shaped support (4) and a solidified makeup product (8A) arranged in relief on the said support (4) and fastened directly to the support (4), the support (4) extending perimetrally well beyond the solidified makeup product (8A) and being visible during use of the cosmetic product (1), the solidified makeup product (8A) determining, in the area of contact with the support (4), a closed perimeter line (21), comprising the following steps:
a. arranging a mould (2) with at least one casting cavity (3A, 3B, 3C, 3D) shaped so as to obtain the final form of at least part of the cosmetic product (1) and having a mouth (23) with a form corresponding to the perimeter line (21);
b. placing the support (4) that will become part of the cosmetic product (1) on top of the mould (2), the said support (4) featuring a plurality of fixing windows (20A, 20B, 20C) arranged inside the perimeter line (21);
c. placing a mask (6) endowed with at least one side containment opening (7) on top of the support (4); and
d. conveying a makeup product (8) into the casting opening (7) in the mask (6) to allow the said product to penetrate and fill the casting cavity (3A, 3B, 3C, 3D) by flowing through both the said opening (7) and the plurality of fixing windows (20A, 20B, 20C);
e. waiting for the solidification of the makeup product (8) inside the mould (6) cavity and removing the mould (2), and
f. removing the mask (6) from the support (4) before or after solidification of the makeup product (8).

## Description

### FIELD OF THE INVENTION

The present invention relates to a method for making a cosmetic product and a cosmetic product made with the said method.

In particular, the invention relates to a cosmetic product for makeup use.

### BACKGROUND ART

The cosmetic product industry, particularly that concerning makeup, has always been in search of innovative products with an eye-catching appearance, also in order to present makeup products whose basic composition is already commonly known in an innovative manner.

Furthermore, over recent times, consumers' attention has shifted towards environmental issues and cosmetic products that can safeguard the environment by providing more recyclable packaging are preferred.

EP 3 669 700 A1, EP 0 038 645 A1, FR 2 704 402 A1 and JP 2002 32908 A describe cosmetic products known in the art.

### SUMMARY OF THE INVENTION

The object of the present invention is to provide a cosmetic product, preferably a makeup product, which is improved with respect to the prior art.

A further object of the invention is to provide a cosmetic product, preferably a makeup product, which has an innovative, eye-catching form.

A still further and non-final object of the present invention is to provide a cosmetic product, preferably a makeup product, which is environmentally friendly and easily recycled.

This and other objects are achieved by means of a cosmetic product, preferably a makeup product, produced according to the technical teachings of the claims annexed hereto.

### BRIEF DESCRIPTION OF THE FIGURES

Further features and advantages of the innovation will become clearer in the following description of a preferred but not exclusive embodiment of the cosmetic product, preferably a makeup product, illustrated - by way of a non-limiting example - in the drawings annexed hereto, in which:
Figure 1 is plan view of a support which is part of the cosmetic product according to the present invention;
Figure 2 is plan view of a mould for the production of the cosmetic product according to the present invention;
Figure 3 is a schematic simplified sectional view, taken along line III-III in Figure 1, when the support is coupled to the mould in Figure 2 and a mask, during production of the cosmetic product according to the invention;
Figure 3A is an enlarged view with multiple details (but also shown schematically) of the part circled in Figure 3;
Figure 4 shows a step in the production of the cosmetic product according to the invention;
Figure 5 shows a final step in the production of the cosmetic product according to the invention, subsequent to the step in Figure 4;
Figure 5A shows a different final step in the production of the cosmetic product according to the invention, also subsequent to the step in Figure 4;
Figure 6 shows the cosmetic product according to the invention, in the finished form and housed in a primary container;
Figure 7 shows, in a plan view, a possible embodiment of the support in Figure 1 which is integrated into a die-cut element also used to make a primary container for the cosmetic product;
Figure 8 is a view of the die-cut element in Figure 1 after the cosmetic product has been made thereupon and the die-cut element has been partially folded;
Figure 9 is a view of the die-cut element in Figure 8 completely folded so as to form a cosmetic product which is integrated into the primary container thereof;
Figure 10 is a plan view of a part of the support 4, at a fastening opening, in one possible configuration thereof;
Figure 10A is a perspective view of the same part of the support as in Figure 10;
Figure 11 is a plan view of a part of the support 4, at a fastening opening, in a further possible configuration thereof;
Figure 11A is a perspective view of the same part of the support as in Figure 11;
Figure 12 is a plan view of a part of the support 4, at a fastening opening, in a still further possible configuration thereof; and
Figure 12A is a perspective view of the same part of the support as in Figure 12.

### DETAILED DESCRIPTION OF THE INVENTION

With reference to the figures stated, reference number 1 is used to denote, as a whole, a cosmetic product, particularly a makeup product.

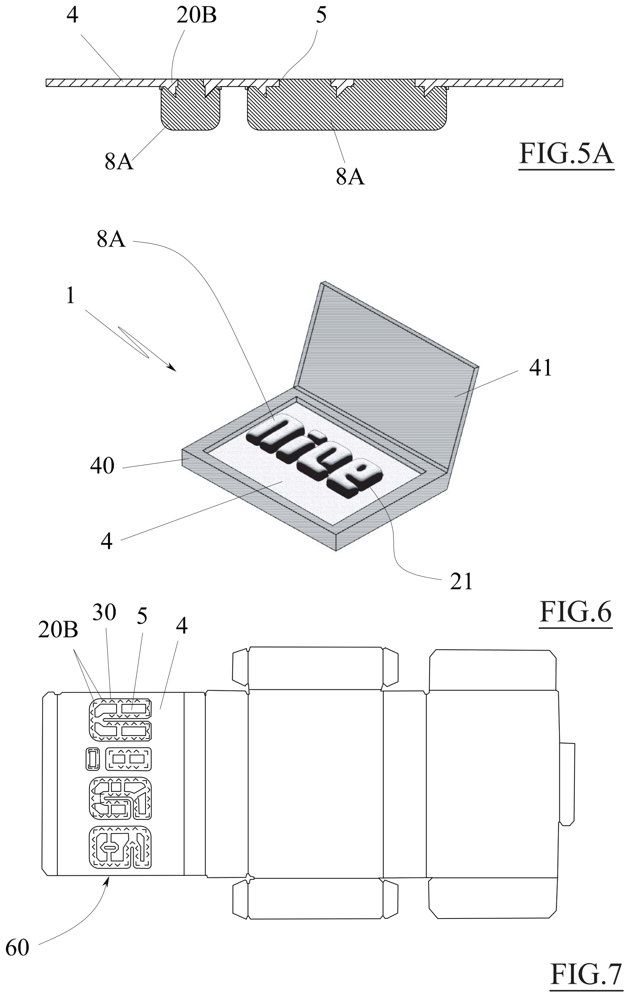

The cosmetic product 1 according to the present invention is clearly visible in Figure 6, where the said product is housed inside a primary container, i.e. a box 40 equipped with a lid 41.

It should be noted that the cosmetic product 1 comprises a plate-shaped support 4 and a solidified makeup product 8A arranged in relief on the support 4.

The solidified makeup product 8A is arranged on the support 4 forming, for example, four stylised letters, i.e. an 'n', an 'i', a 'c' and an 'e'.

It should be noted that the letters are in high relief with respect to the support. This is precisely one of the primary characteristics of the cosmetic product 1 according to the present invention, i.e. the characteristic of allowing the production of high-relief features with respect to conventional cosmetic products.

A further important characteristic is that the solidified makeup product 8A is fastened directly to the support 4, therefore without the interposition of anything else.

It can be noted that the support 4 is plate-shaped (base), therefore with dimensions H and L greater than the third dimension (i.e. thickness, S).

For example, the ratio between the width H and the thickness S is greater than 15 to 1.

The support 4 has, for example, a rectangular conformation, but other shapes are possible, such as, for example, a square, a circle, an ellipse, a triangle etc., depending on the desired aesthetic effect.

The support 4 may be glued (or otherwise secured) to the box 40. In the examples below, however, alternatives to gluing will be shown.

Preferably the support 4 can be cardboard, which may also be decorated by means of printing.

Alternatively, there can also be a fabric element, for example a prestigious fabric, which may also be highly embellished with printing or decorations.

The support can also be a plastic plate, which may be, for example, printed or screen-printed.

Returning to Figure 6, it can be noted that the support 4 extends perimetrally well beyond the solidified makeup product 8A and is therefore visible during use (conventional or for display purposes) of the said cosmetic product 1.

Consequently, any decorations present on the support 4 are also visible.

The solidified makeup product 8A determines, in the zone in which the said product comes into contact with the support 4, a closed perimeter line 21.

In the example, there is one closed perimeter line 21 for each letter (for example for the 'i') or for each part of a letter.

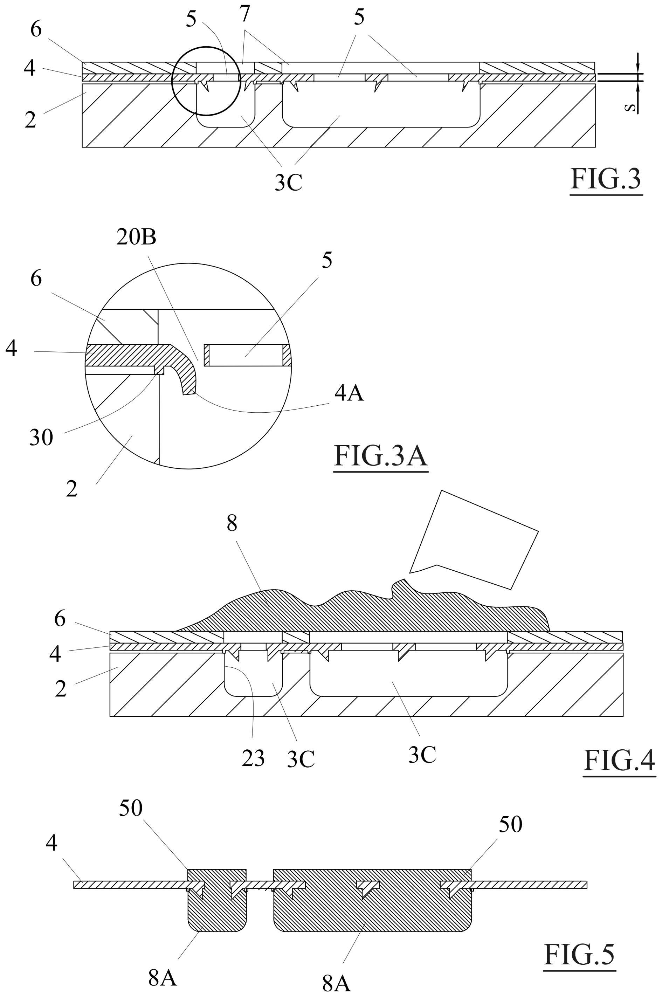

The method for producing the cosmetic product 1 will now be outlined with particular reference to Figures 3 to 5.

Initially (step a.), a mould 2 is provided with at least one casting cavity 3A, 3B, 3C, 3D shaped so as to obtain the final form of at least part of the cosmetic product 1 and having a mouth 23 whose shape corresponds to the perimeter line 21. In Figure 4 and in the following figures, which are sectional views, focus is concentrated on the letter 'I', therefore on the cavities 3C in the mould 2.

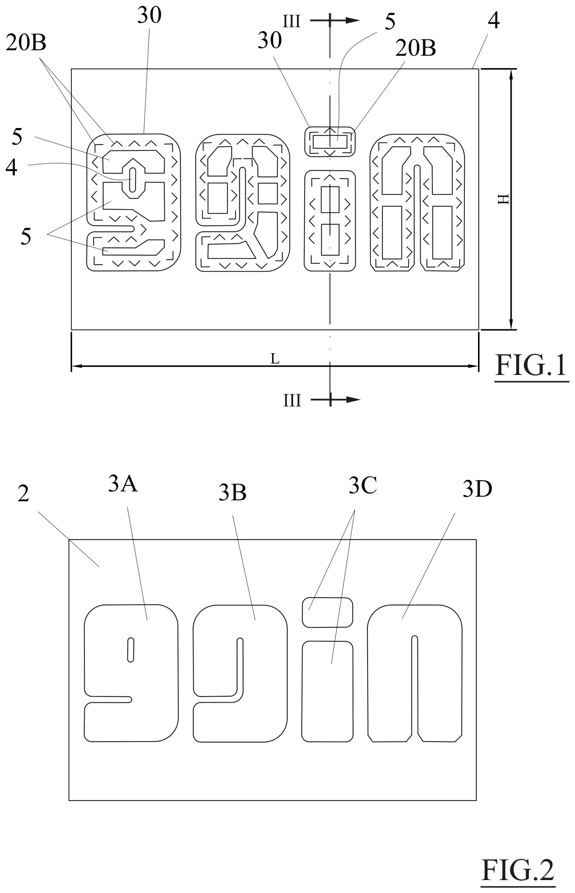

In Figure 2, the mould 2 is shown in a plan view, from the casting side. For illustrative purposes, in the said mould, cavities can be noted with a shape corresponding to the letters visible on the finished product in Figure 6.

For example, the mould 2 features four cavities, each one having the negative conformation of the corresponding letter. The cavity 3C is formed of two separate cavities, one for the downstroke of the 'i' and one for the dot above the 'i'.

The mould 2 can be made of any commonly known material which can be used to create moulds, such as, for example, metal. It has been found that a silicone mould 2 is particularly suitable for the purpose.

In a subsequent step (step b.), the support 4 which will become part of the cosmetic product 1 is placed on top of the mould 2.

As is visible in Figure 1, the support 4 features a plurality of fixing windows 20A, 20B, 20C arranged inside the perimeter line 21. The usefulness of the fixing windows, and likewise the possible conformation thereof, will emerge more clearly later on.

In practice, the support 4 is brought into close contact with the mould 2, centred with respect thereto.

For example, the mouth 23 of each casting cavity present in the mould 2 can be aligned with the respective perimeter line 21 on the support 4, which can also be accentuated by a creasing 30 in relief with respect to the support.

The creasing 30 can have the function of sealing between the mould 2 and the support 4.

Therefore, the said creasing can be configured to rest on the edges of each cavity (as shown in the Figure 3A) or can penetrate the casting cavity, also at the edge thereof.

Obviously, the creasing 30 can also be totally external to the cavity of the mould 2, acting therefore as a side containment seal.

In a subsequent step (step c.), a mask 6 equipped with at least one side containment opening 7 is placed on top of the support 4.

The opening 7 can have a perimetral shape which essentially corresponds to each perimetral line 21, and the mask 6 is placed on the top of the support 4 so that the opening 7 is aligned with the mouth of the cavity 3A, 3B, 3C.

It should be noted that the mask 6, from a plan view, may have an identical conformation to that shown in Figure 2, therefore, essentially like the plan view of the mould 2.

At the end of the operations stated above (whose order can obviously be reversed or changed provided that the pre-casting configuration in Figure 3 is obtained), the method proceeds with step d., wherein a makeup product 8 is conveyed into the opening 7 in the mask 6 to make the said product penetrate, flowing through both the said opening 7 and the plurality of fixing windows 20A, 20B, 20C into and filling the casting cavity 3A, 3B, 3C, 3D.

The makeup product 8, in this operation, is rendered malleable or castable, for example thanks to a high solvent content (in the case, for example, of the formulation for a baked product) or thanks to heating (in the case, for example, of an anhydrous product, such as, for example, a lipstick or the like).

The step describe above, the aim of which is essentially to fill the cavity with the makeup product, can be carried out either by casting the product 8 on top of the opening 7 in the mask 6 and consequently obtaining gravity filling (perhaps with the aid of a scraper or spatula), or by pressure-feeding the product 8 into the opening 7 in the mask 6.

It must be said that the opening 7 in the mask can also be single, for example, to make the product in a single colour (in the case of the example, with all the letters in the same colour). Therefore, the opening 7 will be configured to fill all the cavities in a single step.

Alternatively, it is possible that the opening 7 copies the profile of each cavity, thereby allowing each cavity to fill individually.

There can also be multiple masks 6 envisaged, each one with an opening which allows just one or multiple cavities to be filled.

The mask 6 can also be directly integrated into a dispensing mouth from which the cosmetic product 8 is fed, under light pressure, to the cavity or cavities.

Once the cavity filling has ended, it is necessary to wait for the makeup product 8 to solidify in the cavity or cavities in the mould 2.

The solidification step can take place differently depending on the cosmetic product involved.

In the case of a baked product, at least the mould and the support can be positioned in an oven that facilitates evaporation of the solvent present in the makeup product 8, so as to obtain the solidified makeup product 8A.

In the event that the cosmetic product is wax based, solidification occurs simply by leaving the product to cool. For this purpose, to accelerate solidification, at least the mould 2 and the support 4 can be appropriately cooled.

Once the solidified makeup product 8 has been obtained, the mould 2 can be removed and also the mask if the latter has not already been removed.

Therefore, the mask 6 can be removed before or after solidification of the makeup product, as the case may be.

It can be noted, from Figure 5, that the solidified makeup product is securely fastened to the support 4. The cosmetic product in Figure 5 is obtained by scraping the makeup product 8 onto the surface of the mask 6, also to facilitate penetration thereof through the opening 7 therein.

The excellent fastening can be due to various 'actions' that will be described below.

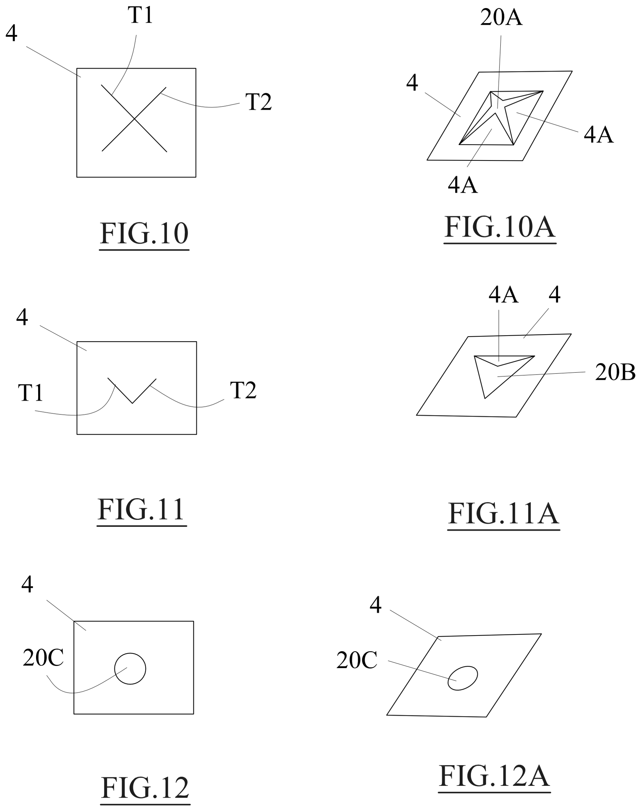

If the fixing windows are configured as in Figure 10 or 11 (therefore, the said windows are cuts made without removing material from the support 4), the edges of the cut fold in the same direction as the casting, creating protruding appendices 4A with respect to the support 4 (as visible in Figure 5) that are embedded into the solidified makeup product 8A, increasing the 'grip' surface between the product and the support 4.

Obviously, the length of the protruding appendices 4A (once the size of the cuts has been determined) can be determined according to the makeup product 8 used (longer for a baked product, shorter for an anhydrous product).

In the configuration in Figure 5, the fixing windows also allow fastening by means of a further action.

It should be noted that, here, the support 4 is in practice sandwiched between the part of the solidified makeup product 8A (at the bottom in Figure 5) which is visible (during use) and a part of the solidified makeup product 8B which is not visible.

The formation of the part of the solidified makeup product 8A that is not visible is due to the fact that the mask 6 has its own thickness, which generates, therefore, the part of the solidified makeup product shown with reference number 50, after the makeup product 8 is scraped or spread over the surface of the mask 6.

For this reason, the fixing windows can also be simply configured as shown for the fixing windows 20C in Figure 12, therefore, by removing material from the support 4.

It should be noted that the peripheral form of the fixing windows 20C in Figure 10 is provided for illustrative purposes only. The said windows can have other forms, such as, for example, square, rectangular, etc.

If, however, the scraping takes place after removal of the mask 6 and therefore the support 4 itself is scraped, the configuration obtained is that shown in Figure 5A; in this case it is preferable that the fixing windows are of the type created without removing material from the support (therefore simply cuts T1, T2).

According to one aspect of the invention, the support 4 can feature a plurality of casting windows 5, each one with a noticeable larger surface area than each of the fixing windows 20A, 20B, 20C, configured to allow the makeup product 8 to flow freely into the casting cavity.

This considerably facilitates filling of the mould cavity or cavities.

Advantageously, the fixing windows 20A, 20B, 20C can be arranged around the perimeter line 21, apart therefrom, and can also surround (at least partially) the casting windows.

Furthermore, as already mentioned above, the fixing windows 20A, 20B (shown in Figures 10 and 11) can be made by making at least two incident cuts T1, T2 in the support 4, so that, at least while the makeup product 8 is flowing through the fixing window 20A, 20B (during casting), one edge of the support 4, at the at least two cuts T, is moved in the casting direction so as to protrude from the support 4 itself, forming the appendix 4A, which is embedded in the solidified makeup product 8A, securing the latter to the support 4.

Advantageously, the cuts T are essentially linear, and/or arranged so as to form a V or an X, as visible in Figures 10 or 11.

To conclude the description, it must be emphasised that the invention also relates to a cosmetic product made using the method described above.

Figure 7 shows the die-cut element 60 with the support 4 in Figure 1 added thereto.

The embodiment in Figure 7 is particularly effective since, advantageously, the peripheral edges and the fold lines of the die-cut element can both be made in a single step, by die-cutting, but also the fixing windows 20B, any casting windows 5, and any creasing 30, can all also be made in/on the same piece of cardboard.

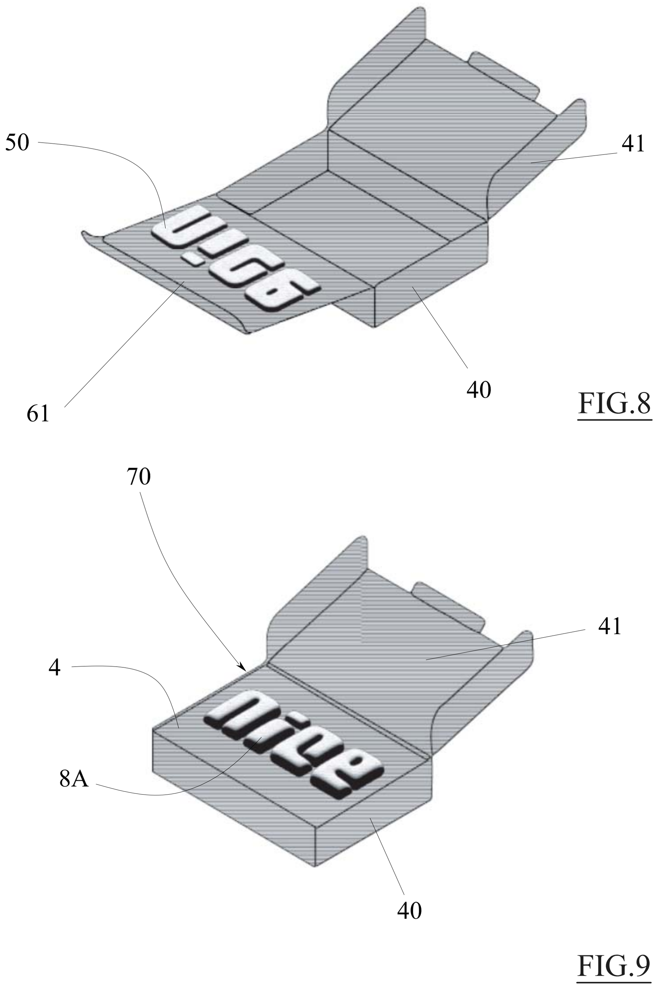

After carrying out the steps of the method described above on part of the support 4, the die-cut element can be folded to form the box shown in Figure 8.

By tucking the protruding flap 61 inside the box, the contents of Figure 9 are obtained, i.e. the finished product already inside the primary packaging.

In this case, the support 4 is integrated directly into the primary container of the said cosmetic product, providing a highly innovative and environmentally friendly packaging system.

Various embodiments of the innovation have been disclosed herein, but further embodiments may also be conceived using the same innovative concept.

## Claims

1. Method for producing a cosmetic product (1) comprising a support (4) conformed as a plate and a solidified makeup product (8A) arranged in relief on the support (4) itself and directly attached to the support (4), the support (4) extending perimeterally well beyond the solidified makeup product (8A) and being visible during the use of the cosmetic product (1), the solidified makeup product (8A) defining, in the area of contact with the support (4), a closed perimeter line (21), comprising the steps of:
a. preparing a mold (2) with at least one casting cavity (3A, 3B, 3C, 3D) shaped to obtain the final shape of at least part of the cosmetic product (1) and having a mouth (23) of a shape corresponding to the perimeter line (21),
b. superimposing on the mold (2) the support (4) that that will be part of the cosmetic product (1), the support (4) having a plurality of fixing windows (20A, 20B, 20C) arranged internally to the perimeter line (21);
c. superimposing on the support (4) a mask (6) having at least one lateral containment opening (7); and
d. conveying a make-up product (8) into the lateral containment opening (7) of the mask (6) to make it penetrate, passing both through said opening (7) and through the plurality of fixing windows (20A, 20B, 20C), inside the casting cavity (3A, 3B, 3C, 3D), filling it;
e. waiting for the solidification of the makeup product (8) in the mold cavity (6) and removing the mold (2) and,
f. before or after the solidification of the makeup product (8), removing the mask (6) from the support (4).

2. Method according to claim 1, wherein the lateral containment opening (7) has a perimetral shape substantially corresponding to the perimeter line (21), and the mask (6) is overlaid on the support (4) so that the opening (7) is aligned with the casting cavity (3A, 3B, 3C) mouth (23).

3. Method according to claim 1, wherein the support (4) provides a plurality of pouring windows (5), of significantly greater area than each of the fixing windows (20A, 20B, 20C), configured to allow free passage of the makeup product (8) into the casting cavity.

4. Method according to claim 1, wherein the fixing windows (20A, 20B, 20C) are arranged along said perimeter line (21) and offset therefrom.

5. Method according to claim 1, wherein at least some of the fixing windows (20C) are through openings made by removing part of the support (4).

6. Method according to claim 1, wherein at least some of the fixing windows (20A, 20B) are made by making at least two incident cuts (T1, T2) in the support (4), so that, at least in the passage of the makeup product (8) through each fixing window (20A, 20B) an appendix (4A) of the support (4) at the at least two cuts (T) is dragged in the casting direction so as to protrude from the support (4) itself and being incorporated into the solidified makeup product (8A).

7. Method according to the preceding claim, wherein the cuts (T1, T2) are substantially linear, and/or arranged in a V shape, or in an X shape.

8. Method according to claim 1, wherein, at said perimeter line (21), the support (4) provides a raised creasing (30).

9. Method according to claim 1, wherein the cosmetic product (8) is scraped over the mask (6) to help the penetration of the cosmetic product into the opening (7).

10. Method according to claim 1 wherein, after removal of the mask (6) the cosmetic product (8) is scraped over the support (4).

11. Method according to claim 1, wherein the support (4) is made in one piece with a die cut (60) which, when folded, forms a primary container (70) of the cosmetic product.

12. A cosmetic product made by the method of one or more of claims 1-11.
